(19) Europäisches Patentamt
European Patent Office
Office européen des brevets

(11) **EP 3 746 850 B1**

(12) **EUROPÄISCHE PATENTSCHRIFT**

(45) Veröffentlichungstag und Bekanntmachung des Hinweises auf die Patenterteilung:
**23.02.2022  Patentblatt 2022/08**

(21) Anmeldenummer: **19703652.8**

(22) Anmeldetag: **28.01.2019**

(51) Internationale Patentklassifikation (IPC):
**G06N 3/04** *(2006.01)*      **G06N 3/08** *(2006.01)*
**G06N 7/00** *(2006.01)*      **G06N 20/10** *(2019.01)*
**G05B 17/02** *(2006.01)*

(52) Gemeinsame Patentklassifikation (CPC):
**G05B 17/02; G06N 3/0472; G06N 3/084;
G06N 7/005; G06N 20/10**

(86) Internationale Anmeldenummer:
**PCT/EP2019/052026**

(87) Internationale Veröffentlichungsnummer:
**WO 2019/149664 (08.08.2019 Gazette 2019/32)**

(54) **VERFAHREN ZUM ERMITTELN EINES ZEITLICHEN VERLAUFS EINER MESSGRÖSSE, PROGNOSESYSTEM, AKTORSTEUERUNGSSYSTEM, VERFAHREN ZUM TRAINIEREN DES AKTORSTEUERUNGSSYSTEMS, TRAININGSSYSTEM, COMPUTERPROGRAMM UND MASCHINENLESBARES SPEICHERMEDIUM**

METHOD FOR ASCERTAINING A TIME CHARACTERISTIC OF A MEASURED VARIABLE, PREDICTION SYSTEM, ACTUATOR CONTROL SYSTEM, METHOD FOR TRAINING THE ACTUATOR CONTROL SYSTEM, TRAINING SYSTEM, COMPUTER PROGRAM AND MACHINE-READABLE STORAGE MEDIUM

PROCÉDÉ DE DÉTERMINATION D'UNE VARIATION TEMPORELLE D'UNE GRANDEUR DE MESURE, SYSTÈME DE PRONOSTIC, SYSTÈME DE COMMANDE D'ACTIONNEUR, PROCÉDÉ D'APPRENTISSAGE DU SYSTÈME DE COMMANDE D'ACTIONNEUR, SYSTÈME D'APPRENTISSAGE, PROGRAMME D'ORDINATEUR ET SUPPORT D'INFORMATIONS LISIBLE PAR MACHINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.01.2018  DE 102018201411**

(43) Veröffentlichungstag der Anmeldung:
**09.12.2020  Patentblatt 2020/50**

(73) Patentinhaber:
 • **Robert Bosch GmbH
  70442 Stuttgart (DE)**
 • **Max-Planck-Gesellschaft zur Förderung der Wissenschaften E.V.
  80539 München (DE)**

(72) Erfinder:
 • **NGUYEN-TUONG, The Duy
  71229 Leonberg (DE)**
 • **DANIEL, Christian
  71229 Leonberg (DE)**
 • **TRIMPE, Sebastian
  72076 Tübingen (DE)**
 • **SCHIEGG, Martin
  70825 Korntal-Muenchingen (DE)**
 • **DOERR, Andreas
  70565 Stuttgart (DE)**

(74) Vertreter: **Banse & Steglich
Patentanwälte PartmbB
Patentanwaltskanzlei
Herzog-Heinrich-Straße 23
80336 München (DE)**

(56) Entgegenhaltungen:
**DE-U1-202017 102 235**

- **STEFANOS ELEFTHERIADIS; THOMAS F.W. NICHOLSON; MARC PETER DEISENROTH; JAMES HENSMAN: "Identification of Gaussian Process State Space Models", ARXIV PREPRINT ARXIV:1705.10888V2, 2017, XP002791073, in der Anmeldung erwähnt**

**Beschreibung**

Stand der Technik

Aus "Variational Gaussian Process State-Space Models", arXiv preprint arXiv:1406.4905v2, 2014, Roger Frigola, Yutian Chan und Carl E. Rasmussen ist ein Verfahren zum variationalen bayes'schen Lernen eines nichtlinearen Zustandsraummodells (Englisch: *state-space model)* mittels spärlicher Gaußprozesse (Englisch: sparse Gaussian processes) bekannt.

Aus "Identification of Gaussian Process State Space Models", arXiv preprint arXiv: 1705.10888v2, 2017, Stefanos Eleftheriadis, Thomas F.W. Nicholson, Marc Peter Deisenroth und James Hensman sowie "A flexible state space model for learning nonlinear dynamical systems", arXiv preprint arXiv:1603.05486v2, 2017, Andreas Svensson und Thomas B. Schön sind weitere Verfahren zum Lernen von Zustandsraummodellen bekannt.

Die Druckschrift DE 20 2017 102235 U1 offenbart ein Trainingssystem zum Trainieren eines Aktorsteuerungssystems zum Steuern eines Aktors. Es wird ein Beobachtungswert empfangen, der einen Zustand eines Aktorsystems charakterisiert. Ein erster Ausgabewert eines ersten maschinellen Lernsystems wird abhängig von dem Beobachtungswert ermittelt, wobei der erste Ausgabewert wenigstens einen Teil des Beobachtungswerts charakterisiert. Es wird ein zweiter Ausgabewert eines zweiten maschinellen Lernsystems ermittelt, wobei der zweite Ausgabewert eine Wahrscheinlichkeit charakterisiert, dass der Beobachtungswert derart manipuliert wurde, dass der erste Ausgabewert den wenigstens einen Teil des ersten Beobachtungswerts nicht korrekt charakterisiert. Der Aktor wird abhängig von dem ermittelten ersten Ausgabewert und dem ermittelten zweiten Ausgabewert angesteuert.

Vorteil der Erfindung

Das Verfahren mit den Merkmalen des unabhängigen Anspruch 1 hat demgegenüber dem Vorteil, dass eine besonders effiziente und robuste Methode ist, nichtlineare Zustandsraummodelle auch bei hochdimensionalen latenten Zustandsräumen zu lernen.

Vorteilhafte Weiterbildungen sind Gegenstand der unabhängigen Ansprüche.

Offenbarung der Erfindung

In einem ersten Aspekt betrifft die Erfindung ein Verfahren zum Ermitteln eines zeitlichen Verlaufs einer Messgröße y, die durch einen Aktor einstellbar ist, wobei auf den Aktor ein zeitlicher Verlauf einer Steuergröße u aufgebracht wird.

Das Ermitteln erfolgt mittels eines Gaußprozess-Zustandsmodells (Englisch: *Gaussian process state space model,* kurz *GP-SSM),* welches ein Verhalten des Aktors beschreibt. Hierbei wird eine Steuergröße des Aktors abhängig von einer parametrierbaren Familie von Funktionen q($x_{1:T}$, $f_{2:T}$, z) ermittelt.

Zustandsraummodelle, auch *Zustandsmodelle* genannt, beschreiben das Verhalten eines Systems mittels einer latenten Variable x unter einem Übergangsmodell f und Prozessrauschen (Englisch: *process noise)* $\epsilon_x$. Abhängig von der latenten Variable x wird eine Messgröße y mittels einer Beobachtungsfunktion g mit Messrauschen $\epsilon_y$ ermittelt. Mittels eines Subscripts "t" kann wie üblich eine zeitliche Entwicklung beschrieben werden. Ein Zustandsraummodell ist dann gegeben als

$$x_{t+1} = f(x_t, u_t) + \epsilon_x$$
$$y_t = g(x_t) + \epsilon_y \tag{1}$$

Mit Gaußprozessen können Verteilungen über Funktionen dargestellt werden. Dies erlaubt es, a-priori-Annahmen über das Verhalten eines Systems zu berücksichtigen. Hierbei wird für einen Satz an Beobachtungen X = [$x_1$, ..., $x_N$] die korrespondierenden Funktionswerte $\boldsymbol{f}$ = [$f(x_1)$, ..., $f(x_N)$] als gemeinsam Gaußverteilt angenommen, also

$$p(\boldsymbol{f}|X) = \mathcal{N}(\boldsymbol{f}|m_X, K_{X,X}), \tag{2}$$

Mit Mittelwertvektor mx mit Einträgen $m_i = m(x_i)$ und einer Kovarianzmatrix Kx,x mit Elementen $K_{ij} = k(x_i, x_j)$. $\mathcal{N}$ bezeichnet wie üblich eine Normalverteilung. Die Einträge des Mittelwertvektors mx können beispielsweise gleich Null gewählt werden. Die Funktion k($x_i$, $x_j$) kann mit geeigneten Hyperparametern $\sigma_f^2$, $\Lambda = diag(l_1^2 ... l_N^2)$ z.B. gegeben

sein durch $k(x_i, x_j) = \sigma_f^2 \exp\left(-\frac{1}{2}(x_i - x_j)^T \Lambda^{-1}(x_i - x_j)\right)$.

Bei gegebenen Funktionswerten **f** an Beobachtungen **X** lässt sich die bedingte Wahrscheinlichkeitsverteilung an einer neuen Beobachtungsstelle x* schreiben als

$$p(f^*|x^*, \boldsymbol{f}, \boldsymbol{X}) = \mathcal{N}(f^*|\mu, \sigma^2) \qquad (3)$$

schreiben, mit $\mu = m_{x^*} + k_{x^*,X} K_{X,X}^{-1}(f - m_X)$, $\sigma^2 = k_{x^*,x^*} - k_{x^*,X} K_{X,X}^{-1} k_{X,x^*}$, wobei $k_{A,B}$ einen Skalar bzw. Vektor von Kovarianzen für jedes Paar von Elementen in A, B bezeichnet. Wichtig ist hier, dass der Kernel so gewählt ist, dass die Funktionen $\mu$, $\sigma$ nach x* differenzierbar sind.

Mit der Abkürzung $\hat{x}_t = (x_t, u_t)$ lässt sich das Übergangsmodell auch schreiben als $f_{t+1} = f(\hat{x}_t)$. Eine Zeitreihe von beobachteten Messgrößen y von einem Zeitpunkt a bis zu einem späteren Zeitpunkt b wird als $y_{a:b}$ abgekürzt (analog für andere Größen).

Vorteilhafterweise kann der beschreibende Gaußprozess als spärlicher Gaußprozess implementiert sein. Hierzu können induzierende Gaußprozess-Zielzustände (Englisch: *inducing Gaussian process targets)* z=[z$_1$,... ,z$_P$] an vorgebbaren pseudo-Eingabepunkten (Englisch: *pseudo input points*) ζ=[ζ$_1$,...,ζ$_P$] vorgesehen sein. Dies verringert den Rechenaufwand der Anpassung der Parameter des Gaußprozesses, insbesondere bei großen Trainingsdatensätzen.

Ein Gaußprozess kann sowohl für das Übergangsmodell f als auch für die Beobachtungsfunktion g gewählt werden. Es ist für die Identifikation der den Gaußprozess charakterisierenden Parameter allerdings hilfreich, ein bekanntes parametrisches Beobachtungsmodell zu wählen, beispielsweise

$$p(y_t|x_t) = \mathcal{N}(y_t|g(x_t), R) \qquad (4)$$

mit diagonaler Kovarianzmatrix $R = \mathrm{diag}(\sigma_{y,1}^2, \dots, \sigma_{y,D_y}^2)$ und Beobachtungsfunktion

$$g(x_t) = C x_t, \qquad (5)$$

wobei die Matrix C so gewählt ist, dass sie die $D_y$ ersten Einträge der latenten Variable $x_t$ auswählt, also $C = [I, 0] \in \mathbb{R}^{D_y, D_x}$, wobei I die Einheitsmatrix ist. Mit $D_x$ wird hierbei die Dimension des Raums der latenten Variablen x bezeichnet, mit $D_y$ die Dimension des Raums der Messgröße y. Dieses Modell ist besonders passend, wenn $D_y < D_x$ ist, was üblicherweise in Systemen mit einer begrenzten Anzahl physikalischer Sensoren, beispielsweise bei Robotern, der Fall ist. Die Beobachtungsfunktion g kann aber auch durch eine andere parametrische, differenzierbare Abbildung gegeben sein. Für hochdimensionale Beobachtungsräume, also großes $D_y$, kann beispielsweise als Beobachtungsfunktion g auch eine andere differenzierbare Funktion, z.B. ein neuronales Netzwerk, eingesetzt werden.

Die gemeinsame Verteilungsfunktion der Variablen des GP-SSM lässt sich dann schreiben als

$$p(y_{1:T}, x_{1:T}, f_{2:T}, z) = \prod_{t=1}^{T} p(y_t|x_t)$$
$$\times \prod_{t=2}^{T} p(x_t|f_t) p(f_t|\hat{x}_{t-1}, z)$$
$$\times p(x_t) p(z). \qquad (6)$$

Hierbei bezeichnet $p(f_t|\hat{x}_{t-1}, z) = \prod_{d=1}^{D_x} p(f_{t,d}|\hat{x}_{t-1}, z_d)$ und $z = [z_1, \dots, z_{D_x}]$.

Das Prozessrauschen kann als $p(x_t|f_t) = \mathcal{N}(x_t|f_t, Q)$ mit diagonaler Kovarianz $Q = \mathrm{diag}(\sigma_{x,1}^2, \dots, \sigma_{x,D_x}^2)$ gewählt werden. Die initiale Verteilung der latenten Zustände $p(x_1)$ ist unbekannt. Die Übergangsdynamik wird für jede latente Dimension d unabhängig durch $p(f_{t,d}|\hat{x}_{t-1}, z_d) p(z_d)$ beschrieben. Diese Wahrscheinlichkeit kann durch die spärliche Gauß'sche a-posteriori Wahrscheinlichkeitsverteilung

$$p\left(f_{t,d}|\hat{x}_t, z_d, \zeta_d\right) \approx p(f_{t,d}|\hat{x}_t, f, X) \tag{7}$$

analog zu Gleichung (3) gewählt werden. Die a-priori-Wahrscheinlichkeitsverteilung $p(z_d)$ der induzierenden Zielzustände $z_d$ kann ebenfalls normalverteilt mit geeigneten Parametern $m_\zeta$, $K_{\zeta,\zeta}$ gewählt werden:

$$p(z_d) = \mathcal{N}(m_{\zeta_d}, K_{\zeta_d,\zeta_d})$$

(8)

Die Integration, die notwendig ist, um eine log-likelihood oder eine a-posteriori Wahrscheinlichkeitsverteilung für das durch Gleichung (6) gegebene Modell zu ermitteln, ist nur sehr schwer lösbar. Daher gibt es Ansätze, eine Variationsfunktion zu verwenden.

Aus "Recurrent Gaussian processes", arXiv preprint arXiv:1511.06644.v6, 2016, Cesar Lincoln C. Mattos, Zhenwen Dai, Andreas Damianou, Jeremy Forth, Guilherme A. Barreto, Neil D. Lawrence ist beispielsweise bekannt, eine sogenannte *mean field approximation* für die latenten Zustände $x_{1:T}$ einzuführen. Hierbei wird die a-posteriori Wahrscheinlichkeitsverteilung p($x_{1:T}$, $f_{2:T}$, z | $y_{1:T}$) eines durch Gleichung (6) gegebenen Modells durch eine Familie von faktorisierten Näherungsfunktionen q der Form

$$q(x_{1:T}, f_{1:T}, z) = \prod_{t=1}^{T} q(x_t) \prod_{d=1}^{D_x} q(z_d) \prod_{t=1}^{T} p(f_t|z, x_t) \tag{9}$$

approximiert. Durch Variation der diese Familie parametrierenden Parameter kann dann versucht werden, die tatsächliche a-posteriori Wahrscheinlichkeitsverteilung bestmöglich anzunähern.

Um die im Modell (6) vorhandenen zeitlichen Korrelationen zwischen Zuständen besser beizubehalten, kann statt des oben genannten Ansatzes die parametrierbare Familie von Funktionen q($x_{1:T}$, $f_{2:T}$, z) derart gewählt werden, dass eine zeitliche Abhängigkeit der zeitlich aufeinander folgenden latenten Zustände x des Aktors erhalten bleibt. D.h. die Abhängigkeit eines zeitlich nachfolgenden latenten Zustand $x_t$ des Aktors (der beispielsweise mit einer Übergangsfunktion ermittelt wurde) von einem zeitlich vorhergehenden, insbesondere unmittelbar vorhergehenden latenten Zustand x des Aktors und einer zeitlich vorhergehenden, insbesondere unmittelbar vorhergehenden Steuergröße u des Aktors soll gleich der entsprechenden Abhängigkeit des Gaußprozess-Zustandsmodell, insbesondere gemäß Gleichung (6), sein. D.h., die durch den Term $p(f_t|\hat{x}_{t-1})$ bzw. (im Fall der spärlichen Formulierung) $p(f_t|\hat{x}_{t-1}, z)$ in Gleichung (6) formulierte Abhängigkeit soll in der parametrierbaren Familie von Funktionen q($x_{1:T}$, $f_{2:T}$, z) ebenfalls so erhalten bleiben.

Insbesondere kann vorgesehen sein, dass die parametrierbare Familie von Funktionen q($x_{1:T}$, $f_{2:T}$, z) eingerichtet ist, eine a-posteriori Wahrscheinlichkeitsverteilung p($x_{1:T}$, $f_{2:T}$, z | $y_{1:T}$) für einen ermittelten Trainingsdatensatz $y_{mess}$ möglichst gut zu approximieren.

Der Trainingsdatensatz $y_{mess}$ wird vorteilhafterweise ermittelt, indem der Aktor mit einem vorgebbaren Trainingsverlauf $u_{1:n}$ der vorgebbaren Steuergröße u angesteuert wird und ein sich ergebender zeitlicher Trainingsverlauf $y_{1:n}$ der Messgröße y ermittelt wird. Der Trainingsdatensatz $y_{mess}$ kann dann durch ($y_{1:n}$, $u_{1:n}$) gegeben sein.

Vorteilhafterweise die Abhängigkeit der parametrierbaren Familie von Funktionen q($x_{1:T}$, $f_{2:T}$, z) von einem initialen latenten Zustands $x_1$ des Aktors durch einen Faktor gegeben ist, der von diesem initialen latenten Zustand $x_1$ abhängt (insbesondere nur von diesem abhängt), wobei dieser Faktor durch eine parametrierbare Variationsfunktion q($x_1$), insbesondere durch eine Normalverteilung $N(m_{x1}, S_{x1})$ gegeben ist.

Alternativ oder zusätzlich kann eine Abhängigkeit der parametrierbaren Familie von Funktionen q($x_{1:T}$, $f_{2:T}$, z) von Gaußprozess-Zielzuständen $z_d$ jeweils durch einen zweiten Faktor gegeben ist, wobei dieser zweite Faktor jeweils eine zweite parametrierbare Variationsfunktion q($z_d$) ist, die als Argument den jeweiligen Gaußprozess-Zielzustand $z_d$ hat

Hierbei kann die zweite parametrierbare Variationsfunktion q($z_d$) durch eine Normalverteilungsfunktion $N(z_d | m_d, Sd)$, gegeben sein.

Die parametrierbare Familie von Funktionen hat dann die Form

$$q(x_{1:T}, f_{1:T}, z) = \prod_{t=2}^{T} p(x_t | f_t)$$

$$\times \prod_{t=2}^{T} \prod_{d=1}^{D_x} p(f_{t,d} | \hat{x}_{t-1}, z_d) q(z_d)$$

$$\times q(x_1). \hspace{3cm} (10)$$

Die Parameter, mit denen diese parametrierbare Familie von Funktionen parametriert wird, sind dann gegeben durch

- Prozessrauschen
- Sensorrauschen
- Variationsparameter für die induzierenden Gaußprozess-Zielzustände
- Vorgebbare Pseudo-Eingabepunkte
- Kernel-Hyperparameter.

Mit dieser parametrierbaren Familie von Funktionen kann nun vorgesehen sein, dass eine prognostizierte zeitliche Entwicklung des latenten Zustand $\tilde{x}_t$ des Aktors ermittelt wird, indem rekursiv eine Stichprobe der prognostizierten zeitlichen Entwicklung des latenten Zustands zu einem nachfolgenden Zeitpunkt $\tilde{x}_{t+1}$ aus der parametrierbaren Variationsfunktion des prognostizierten latenten Zustands zum nachfolgenden Zeitpunkt gegeben den prognostizierten latenten Zustand zu einem vorherigen Zeitpunkt $q(\tilde{x}_{t+1} | \tilde{x}_t)$ ermittelt wird, wobei die Steuergröße des Aktors abhängig von der prognostizierten zeitlichen Entwicklung des latenten Zustands gewählt wird.

Dies ermöglicht, wie in den Ausführungsbeispielen ausführlicher dargestellt, eine einfache Ermittlung einer unteren variationalen Schranke, die auch als ELBO bekannt ist.

Durch die Markov-Struktur der latenten Zustände x und die spärliche Gaußprozess-Näherung ist die (marginalisierte) approximierte Verteilung des latenten Zustands zu einem Zeitpunkt $t$, $q(x_t)$, bei gegebener Verteilung zu einem vorhergehenden Zeitpunkt $t-1$ konditional unabhängig von vorherigen Zeitschritten. Dies erlaubt das oben beschriebene rekursive Vorgehen.

Es ist allerdings notwendig, einen initialen latenten Zustand $\tilde{x}_1$ vorzugeben. Es ist entweder möglich, dass der initiale latente Zustand $\tilde{x}_1$ der prognostizierten zeitlichen Entwicklung des latenten Zustands fest vorgegeben, insbesondere zufällig vorgegeben wird. Dies ist besonders einfach.

Es ist allerdings auch möglich, dass der initiale latente Zustand $(\tilde{x}_1)$ aus der parametrierbaren Variationsfunktion $q(x_1)$ durch eine Verteilungsfunktion des initialen Zustands $x_1$ gegeben den ermittelten Trainingsdatensatz $q(x_1 | y_{1:n}, u_{1:n})$ ) ermittelt wird, deren charakterisierende Parameter durch Rückpropagation trainiert werden.

In einem weiteren Aspekt betrifft die Erfindung ein Verfahren, bei dem eine optimale Steuergröße $u_{opt}$, mit der der Aktor angesteuert werden kann, abhängig von einem mittels eines der vorgenannten Verfahrens ermittelten Verlaufs der Messgröße y ermittelt wird. Dies ermöglicht auf besonders einfache Weise eine optimale Ansteuerung des Aktors.

In einem weiteren Aspekt betrifft die Erfindung ein Verfahren zum Lernen einer Regelstrategie, was auch unter dem Begriff "policy learning" bekannt ist. Hierbei kann vorgesehen sein, dass wenigstens ein optimaler Parameter $\xi_{opt}$ ermittelt wird. Dieser optimale Parameter $\xi_{opt}$, charakterisiert eine Regelstrategie eines Aktorsteuerungssystems. Dieses ist eingerichtet abhängig von dieser Regelstrategie den Aktor mit einer Steuergröße u anzusteuern. Die Steuergröße u kann also abhängig von der Regelstrategie und damit abhängig vom optimalen Parameter $\xi_{opt}$ ermittelt. Ein sich bei Anwendung der Regelstrategie ergebender zeitlicher Verlauf der Messgröße y wird mittels eines der vorgenannten Verfahrens ermittelt wird, und abhängig von dem so ermittelten Verlauf der Messgröße y wird der wenigstens eine optimale Parameter $\xi_{opt}$ ermittelt.

In einem weiteren Aspekt betrifft die Erfindung ein Verfahren zum Trainieren eines Aktorsteuerungssystems, das eingerichtet ist, eines der vorgenannten Verfahren auszuführen, wobei Parameter, die die parametrierbare Familie von Funktionen $q(x_{1:T}, f_{2:T}, z)$ charakterisieren und/oder deterministische Modell-Parameter derart angepasst werden, dass sie die a-posteriori Wahrscheinlichkeitsverteilung $p(x_{1:T}, f_{2:T}, z | y_{1:T})$ von zumindest zeitlichen Verläufen mindestens des latenten Zustands des Aktors $x_{1:T}$ und der Übergangsfunktion $f_{2:T}$, gegeben den zeitlichen Verlauf der Messgröße $y_{1:T}$ des Aktors für einen ermittelten Trainingsdatensatz möglichst gut approximiert.

Nachfolgend werden Ausführungsformen der Erfindung unter Bezugnahme auf die beiliegenden Zeichnungen näher erläutert. In den Zeichnungen zeigen:

Figur 1    schematisch einen Aufbau von Ausführungsformen der Erfindung;

Figur 2     schematisch einen Aufbau weiterer Ausführungsformen der Erfindung;

Figur 3     in einem Flussdiagramm den Ablauf eines Verfahrens gemäß einem Aspekt der Erfindung;

Figur 4     in einem Flussdiagramm den Ablauf eines Verfahrens gemäß einem weiteren Aspekt der Erfindung;

Figur 5     in einem Flussdiagramm den Ablauf eines Verfahrens gemäß einem noch weiteren Aspekt der Erfindung.

Beschreibung der Ausführungsbeispiele

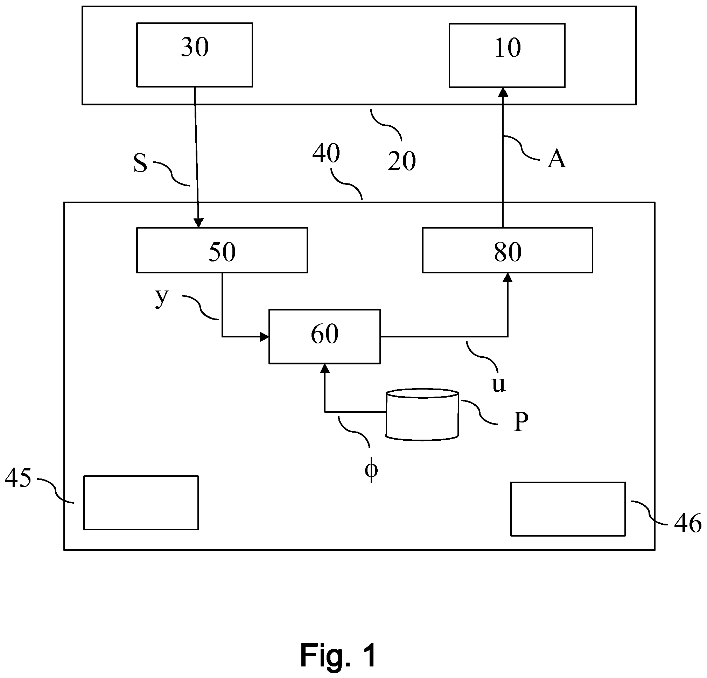

Figur 1 illustriert einen Aufbau möglicher Ausführungsformen der Erfindung. Figur 1 zeigt in einer Ausführungsform einen Aktor 10 in seiner Umgebung 20 in Interaktion mit einem Aktorsteuerungssystem 40. Aktor 10 und Umgebung 20 werden gemeinschaftlich nachfolgend auch als Aktorsystem bezeichnet. In beispielsweise regelmäßigen zeitlichen Abständen wird ein Zustand des Aktorsystems mit einem Sensor 30 erfasst, der auch durch eine Mehrzahl von Sensoren gegeben sein kann. Je ein Sensorsignal S des Sensors 30 wird an das Aktorsteuerungssystem 40 übermittelt. Das Aktorsteuerungssystem 40 empfängt somit eine Folge von Sensorsignalen S. Das Aktorsteuerungssystem 40 ermittelt hieraus eine Folge von Ansteuersignalen A, welches der Aktor 10 empfängt.

Bei dem Aktor 10 kann es sich beispielsweise um einen (teil-)autonomen Roboter, beispielsweise ein (teil-)autonomes Kraftfahrzeug handeln, oder um einen Roboter, der gezielt erkanntes Unkraut in einem Feld bekämpft, beispielsweise ausreißt oder mit entsprechenden Chemikalien besprüht.

Bei dem Sensor 30 kann es sich beispielsweise um einen oder mehrere Videosensoren und/oder einen oder mehrere Radarsensoren und/oder einen oder mehrere Ultraschallsensoren und/oder einen oder mehrere Positionssensoren (beispielsweise GPS) handeln. Alternativ oder zusätzlich kann der Sensor 30 auch ein Informationssystem umfassen, das eine Information über einen Zustand des Aktorsystems ermittelt, wie beispielsweise ein Wetterinformationssystem, das einen aktuellen oder zukünftigen Zustand des Wetters in der Umgebung 20 ermittelt.

In einem anderen Ausführungsbeispiel kann es sich bei dem Aktor 10 um einen Fertigungsroboter handeln, und bei dem Sensor 30 dann beispielsweise um einen optischen Sensor handeln, der Eigenschaften von Fertigungserzeugnissen des Fertigungsroboters erfasst.

In einem weiteren Ausführungsbeispiel kann es sich bei dem Aktor 10 um ein Freigabesystem handeln, welches eingerichtet ist, die Aktivität eines Geräts freizugeben oder nicht. Bei dem Sensor 30 kann es sich beispielsweise um einen optischen Sensor (beispielsweise zur Erfassung von Bild- oder Videodaten) handeln, der eingerichtet ist, ein Gesicht zu erfassen. Der Aktor 10 ermittelt abhängig von der Folge von Ansteuersignalen A ein Freigabesignal, das benutzt werden kann, um abhängig vom Wert des Freigabesignals das Gerät freizugeben. Bei dem Gerät kann es sich beispielsweise um eine physische oder logische Zugangskontrolle handeln. Abhängig vom Wert des Ansteuersignals A kann die Zugangskontrolle dann vorsehen, dass Zugang gewährt wird, oder nicht.

In einem weiteren Ausführungsbeispiel kann es sich bei dem Aktor 10 um einen Teil einer Gebäudesteuerung handeln, beispielsweise um einen Regler einer Heizung.

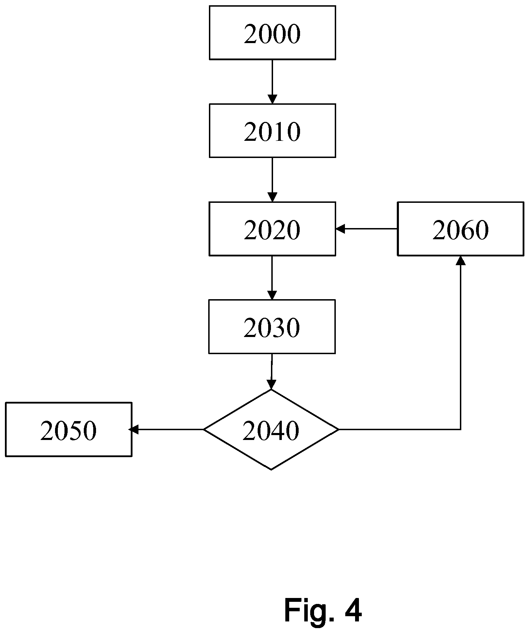

Das Aktorsteuerungssystem 40 empfängt die Folge von Sensorsignalen S des Sensors in einer optionalen Empfangseinheit 50, die die Folge von Sensorsignalen S in eine Folge von Messgrößen y umwandelt (alternativ kann auch unmittelbar je das Sensorsignal S als Messgröße y übernommen werden). Die Messgröße y kann beispielsweise ein Ausschnitt oder eine Weiterverarbeitung des Sensorsignals S sein. Die Messgröße y wird einem maschinellen Lernsystem 60 zugeführt, dessen Funktionsweise unten im Zusammenhang mit Figur 4 näher erläutert wird.

Das maschinelle Lernsystem 60 ermittelt aus den Messgrößen y eine Steuergröße u. Diese Ermittlung erfolgt abhängig von Parametern $\phi$, die in einem Parameterspeicher P abgelegt sind. Diese Parameter $\phi$ können insbesondere Parameter $\xi_{opt}$ umfassen, die eine Regelstrategie des Aktorsteuerungssystems 40 charakterisieren. Der Parameterspeicher P kann in das Aktorsteuerungssystem 40 integriert sein, er kann allerdings auch räumlich getrennt vom Aktorsteuerungssystem 40 ausgebildet sein, und beispielsweise über eine Netzwerkverbindung mit dem Aktorsteuerungssystem 40 verbunden sein. Die Steuergröße u wird einer optionalen Umformeinheit 80 zugeführt, die hieraus Ansteuersignale A ermittelt, welche dem Aktor 10 zugeführt werden.

In weiteren Ausführungsformen umfasst das Aktorsteuerungssystem 40 den Aktor 10.

In weiteren bevorzugten Ausführungsformen umfasst das Aktorsteuerungssystem 40 eine Ein- oder Mehrzahl von Prozessoren 45 und wenigstens ein maschinenlesbares Speichermedium 46, auf dem Anweisungen gespeichert sind, die dann, wenn sie auf den Prozessoren 45 ausgeführt werden, das Aktorsteuerungssystem 40 veranlassen, das Verfahren zum Ansteuern des Aktors 10 auszuführen.

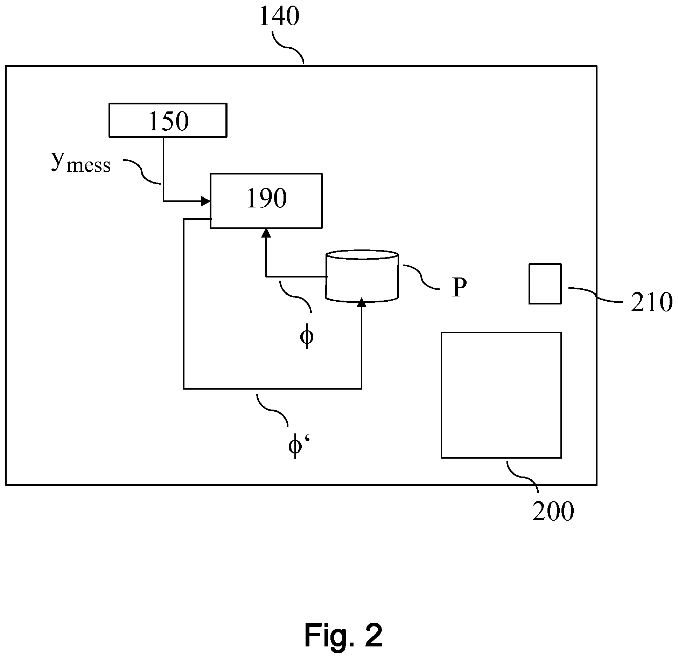

Figur 2 illustriert ein maschinelles Trainingssystem 140, mit der das maschinelle Lernsystem 60 des Aktorsteuerungssystems 40 trainiert werden kann.

Eine Messwertvorrichtung 150 ermittelt einen Trainingsdatensatz $y_{mess}$, der sowohl Steuergrößen u als auch zugehörige Messgrößen y umfasst. Diese können beispielsweise durch Ansteuerung des Aktors 10 mittels der Steuergrößen u und Ermittlung der sich ergebenden Messgrößen y ermittelt worden sein, und auf einem Datenträger (nicht dargestellt) abgespeichert sein, der Teil der Messwertvorrichtung 150 sein kann. Zur Ermittlung des Trainingsdatensatzes y-$_{mess}$ kann die Messwertvorrichtung 150 aus dem Datenträger auslesen.

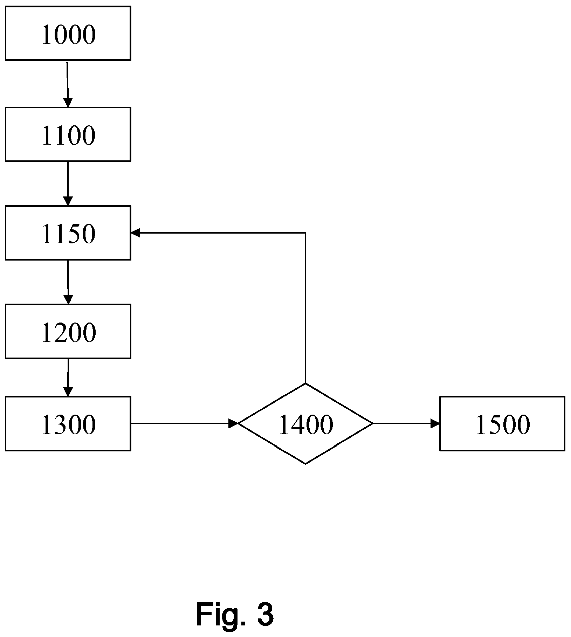

Der Trainingsdatensatz y$_{mess}$ wird einem Trainingsblock 190 zugeführt, der abhängig von dem im Parameterspeicher P gespeicherten Parametern $\phi$ mittels des in Figur 3 illustrierten Verfahrens optimierte Parameter $\phi'$ ermittelt, die im Parameterspeicher P die gespeicherten Parameter $\phi$ ersetzen.

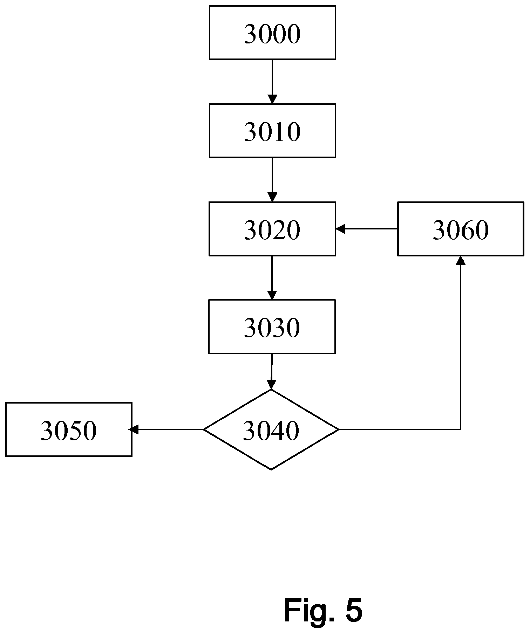

Alternativ oder zusätzlich können mittels des in Figur 5 illustrierten Verfahrens optimierte Parameter $\xi_{opt}$ ermittelt werden, die Teil der optimierten Parameter $\phi'$ sein können, und ebenfalls im Parameterspeicher P hinterlegt werden.

In weiteren bevorzugten Ausführungsformen umfasst das Trainingssystem 140 eine Ein- oder Mehrzahl von Prozessoren 200 und wenigstens ein maschinenlesbares Speichermedium 210, auf dem Anweisungen gespeichert sind, die dann, wenn sie auf den Prozessoren 200 ausgeführt werden, das Trainingssystem 140 veranlassen, das Verfahren zum Trainieren des maschinellen Lernsystems 60 auszuführen.

Figur 3 illustriert eine Ausführungsform eines Verfahrens zum Trainieren des maschinellen Lernsystems 60. Zunächst (1000) werden die Parameter $\phi$ initialisiert und ein Trainingsdatensatz mit zeitlichen Verläufen der Steuergröße u und der Messgröße y bereitgestellt. Die entsprechenden zeitlichen Verläufe werden mit u$_{1:T}$ bzw. y$_{1:T}$ bezeichnet.

Dann (1100) werden diese zeitlichen Verläufe optional in Unterverläufe vorgebbarer Länge T$_{sub}$ zerteilt.

Anschließend wird für den Verlauf bzw. einen oder mehrere der Unterverläufe eine Mehrzahl von Unterverläufen jeweils eine Ein- oder Mehrzahl zugehöriger Trajektorien prognostizierter latenter Variablen $\tilde{x}$ ermittelt. Hierzu wird jeweils zunächst ein initialer prognostizierter latenter Zustand $\tilde{x}_1$ ermittelt, beispielsweise aus der parametrierten Verteilungsfunktion $q(x_1)$ gezogen. Die Parameter dieser Verteilungsfunktion sind dann vorzugsweise ebenfalls Teil der zu optimierenden Parameter $\phi$, da etwaige Fehler, die durch den initialen latenten Zustand hervorgerufen werden, insbesondere bei kurzen zeitlichen Verläufen u.U. nicht hinreichend schnell abklingen. Anschließend erfolgt je nach Länge des zeitlichen Verlaufs eine rekursive Ermittlung der weiteren prognostizierten latenten Zustände $\tilde{x}_t$.

Dann werden jeweils Stichproben $\tilde{x}_t$ aus der Verteilungsfunktion $q(x_t)$ gezogen. Hierzu werden beispielsweise Stichproben $\varepsilon \sim N(0,1)$ gezogen, und anschließend für alle d und alle Zeitpunkte t>1

$$\tilde{x}_{t+1,d} = \mu_d + \epsilon \sqrt{\sigma_d^2(\hat{x}_t, \hat{x}_t) + \sigma_{x,d}^2} \qquad (11)$$

gezogen. Hierbei ist $\hat{x}_t = (\tilde{x}_t, u_t)$.

Anschließend sollen die Parameter $\phi$ derart angepasst werden, dass die Kullback-Leibler-Divergenz $KL(q(x_{1:T}, f_{2:T}, z)\|_p(x_{1:T}, f_{2:T}, z|y_{1:T}))$ minimiert wird, wobei die Länge T im Falle der Unterteilung in Unterverläufe selbstverständlich durch T$_{sub}$ ersetzt wird. Mit der üblichen unteren variationalen Schranke (Englisch: *evidence lower bound,* kurz: *ELBO)* ist das Minimieren dieser KL-Divergenz äquivalent zur Maximierung des ELBO, der gegeben ist durch

$$\mathcal{L}_{GP-SSM} = \mathbb{E}_{q(x_{1:T}, f_{2:T}, z)} \left[ \frac{p(y_{1:T}, x_{1:T}, f_{2:T}, z)}{q(x_{1:T}, f_{2:T}, z)} \right] \qquad (12)$$

$$= \sum_{t=1}^{T} \mathbb{E}_{q(x_t)}[\log p(y_t|x_t)] - \sum_{d=1}^{D} KL(q(z_d)||p(z_d; \zeta_d)). \qquad (13)$$

Daher wird nun (1200) der ELBO gemäß Gleichung (13) geschätzt. Hierzu wird der erste Term der rechten Seite aus Gleichung (13) mittels der prognostizierten zeitlichen Verläufe der latenten Variable x mittels

$$\mathbb{E}_{q(x_t)}[\log p(y_t|x_t)] \approx \frac{1}{N} \sum_{i=1}^{N} \log p(y_t|\tilde{x}_t^i) \qquad (14)$$

geschätzt, wobei N die in Schritt 1100 generierten prognostizierten zeitlichen Verläufe der latenten Variable x bezeichnet.

Ausgehend von dieser stochastischen Ermittlung des ELBO werden Gradienten der Funktion $\mathcal{L}_{GP-SSM}$ ermittelt, und ein stochastischer Gradientenaufstieg der Parameter $\phi$ durchgeführt, um neue Parameter $\phi'$ zu ermitteln (1300).

Nun (1400) wird überprüft, ob ein Konvergenzkriterium erfüllt ist. Ist dies der Fall (1500), ersetzen die neuen Parameter $\phi'$ die im Parameterspeicher P gespeicherten Parameter $\phi$, und das Verfahren endet. Andernfalls wird zurückverzweigt zu Schritt 1150.

Figur 4 illustriert eine Ausführungsform eines Verfahrens zum Ansteuern des Aktors 10. Optional wird zunächst (2000) das in Figur 3 illustrierte Trainingsverfahren durchgeführt. Anschließend (2010-2050) wird eine modellprädiktive Regelung auf der Steuergröße u für einen vorgebbaren Prädiktionshorizont $T_{pred}$ durchgeführt.

Hierzu wird zunächst (2010) ein zeitlicher Verlauf der Steuergröße u generiert. Dann (2020) wird der initiale latenter Zustand $\tilde{x}_1$ ermittelt, beispielsweise zufällig gewählt oder gleich 0 gewählt. Dies ist möglich, da für stabile transiente Dynamik transiente Effekte, die durch einen falsch gewählten initialen latenten Zustand $\tilde{x}_1$ hervorgerufen werden, exponentiell abklingen. Dann wird der latente Zustand $\tilde{x}_{1:T_{pred}}$ beispielsweise mittels Gleichung (11) ermittelt, und mittels der Beobachtungsfunktion g (z.B. durch Gleichung (5) gegeben) auch die Messgröße $y_{1:T_{pred}}$ ermittelt.

Anschließend (2030) wird abhängig vom ermittelten Verlauf der Messgröße $y_{1:T_{pred}}$ eine Kostenfunktion ermittelt.

Dann (2040) wird überprüft, ob ein Konvergenzkriterium der Kostenfunktion erreicht ist. Ist dies der Fall (2050), wird der aktuell ermittelte Verlauf der Steuergröße u als optimale Steuergröße $u_{opt}$ übernommen, und der Aktor 10 wird entsprechend des Verlaufs der optimalen Steuergröße $u_{opt}$ angesteuert.

Ist dies nicht der Fall (2060), wird der Verlauf der Steuergröße u variiert. Beispielsweise kann ein Gradientenabstiegsverfahren eingesetzt werden, wobei die Gradienten beispielsweise numerisch mit Auswertungsschritten analog zu Schritt (2020) ermittelt werden können, oder auch analytisch vorgegeben sein können. Dann wird mit verändertem Verlauf der Steuergröße u zurückverzweigt zu Schritt 2020.

Figur 5 illustriert eine Ausführungsform des Verfahrens zum Ermitteln des Parameters $\xi_{opt}$. Optional wird zunächst (3000) das in Figur 3 illustrierte Trainingsverfahren durchgeführt.

Dann (3010) wird ein initialer Wert der Steuergröße u und ein initialer Wert des Parameters $\xi_{opt}$ generiert. Ebenso wird analog zu Schritt (2020) ein initialer Wert des latenten Zustands x ermittelt. Dann (3020) wird mittels Gleichungen (5) und (11) sowie der durch den Parameter $\xi_{opt}$ charakterisierten aktuellen Regelstrategie ein zeitlicher Verlauf des latenten Zustands u, der Messgröße y und der Steuergröße u ermittelt. Anschließend (4030) wird abhängig vom ermittelten Verlauf der Messgröße eine Kostenfunktion ermittelt.

Dann (3040) wird überprüft, ob ein Konvergenzkriterium der Kostenfunktion erreicht ist. Ist dies der Fall (3050), wird der aktuell ermittelte Parameter $\xi_{opt}$ als optimaler Parameter $\xi_{opt}$ übernommen.

Ist dies nicht der Fall (3060), wird der Parameter $\xi_{opt}$ variiert. Beispielsweise kann ein Gradientenabstiegsverfahren eingesetzt werden. Dann wird mit verändertem Verlauf des Parameters $\xi_{opt}$ zurückverzweigt zu Schritt 3020.

Selbstverständlich können alle Verfahren nicht nur in Software, sondern auch in Hardware implementiert sein, oder in einer Mischform aus Hardware und Software.

## Patentansprüche

1. Verfahren zum Ermitteln eines zeitlichen Verlaufs einer Messgröße (y), die durch einen Aktor (20) einstellbar ist, wobei auf den Aktor (20) ein zeitlicher Verlauf einer Steuergröße (u) aufgebracht wird, wobei das Ermitteln mittels eines Gaußprozess-Zustandsmodells des Verhaltens des Aktors (20) erfolgt, wobei der zeitliche Verlauf der Messgröße (y) des Aktors (20) abhängig von einer parametrierbaren Familie von Funktionen $(q(x_{1:T}, f_{2:T}, z))$ ermittelt wird, wobei in der parametrierbaren Familie von Funktionen $(q(x_{1:T}, f_{2:T}, z))$ eine zeitliche Abhängigkeit eines, insbesondere mit einer Übergangsfunktion (ft) ermittelten, zeitlich nachfolgenden latenten Zustand $(x_t)$ des Aktors (20) von einem zeitlich vorhergehenden latenten Zustand $(_{x_{t-1}})$ des Aktors (20) und einer zeitlich vorhergehenden Steuergröße $(u_{t-1})$ des Aktors (20) gleich der entsprechenden Abhängigkeit des Gaußprozess-Zustandsmodell ist, wobei die entsprechende Abhängigkeit bedeutet, dass die zeitliche Abhängigkeit in der parametrierbaren Familie von Funktionen so erhalten bleibt.

2. Verfahren nach Anspruch 1, wobei die parametrierbare Familie von Funktionen $(q(x_{1:T}, f_{2:T}, z))$ eingerichtet ist, eine a-posteriori Wahrscheinlichkeitsverteilung $(p(x_{1:T}, f_{2:T}, z \mid y_{1:T}))$ von zumindest zeitlichen Verläufen mindestens des latenten Zustands $(x_{1:T})$ des Aktors (20) und der Übergangsfunktion $(f_{2:T})$, bei einem gegebenen zeitlichen Verlauf der Messgröße $(y_{1:T})$ für einen ermittelten Trainingsdatensatz (ymess) möglichst gut zu approximieren.

3. Verfahren nach Anspruch 1 oder 2, wobei die Abhängigkeit der parametrierbaren Familie von Funktionen $(q(x_{1:T}, f_{2:T}, z))$ von einem initialen latenten Zustands $(x_1)$ des Aktors (20) durch einen Faktor gegeben ist, der von diesem initialen latenten Zustand $(x_1)$ abhängt, wobei dieser Faktor durch eine parametrierbare Variationsfunktion (q(xi)), insbesondere durch eine Normalverteilung $(N(m_{x1}, S_{x1}))$ gegeben ist.

4. Verfahren nach einem der Ansprüche 1 bis 3, wobei das Gaußprozess-Zustandsmodell ein spärliches Gaußprozess-Zustandsmodell mit induzierenden Gaußprozess-Zielzuständen (Englisch: *inducing Gaussian process targets*) $(z_1,...,z_P)$ an vorgebbaren pseudo-Eingabepunkten (Englisch: *pseudo inputpoints*) $(\zeta_1,... ,\zeta_P)$ ist.

5. Verfahren nach Anspruch 3 und 4, wobei eine Abhängigkeit der parametrierbaren Familie von Funktionen ($q(x_{1:T}, f_{2:T}, z)$) von Gaußprozess-Zielzuständen ($z_d$) jeweils durch einen zweiten Faktor gegeben ist, wobei dieser zweite Faktor jeweils eine zweite parametrierbare Variationsfunktion ($q(z_d)$) ist, die als Argument den jeweiligen Gaußprozess-Zielzustand ($z_d$) hat, wobei insbesondere die zweite parametrierbare Variationsfunktion ($q(z_d)$) durch eine Normalverteilungsfunktion ($N(z_d \mid m_d, S_d)$) gegeben ist.

6. Verfahren nach einem der Ansprüche 4 bis 5, wobei eine prognostizierte zeitliche Entwicklung des latenten Zustand ($\tilde{x}_t$) des Aktors (20) ermittelt wird, indem rekursiv eine Stichprobe der prognostizierten zeitlichen Entwicklung des latenten Zustands zu einem nachfolgenden Zeitpunkt ($\tilde{x}_{t+1}$) aus der parametrierbaren Variationsfunktion (q) des prognostizierten latenten Zustands zum nachfolgenden Zeitpunkt gegeben den prognostizierten latenten Zustand zu einem vorherigen Zeitpunkt ($q(\tilde{x}_{t+1}|\tilde{x}_t)$) ermittelt wird, wobei der zeitliche Verlauf der Messgröße (y) des Aktors (20) abhängig von der prognostizierten zeitlichen Entwicklung des latenten Zustands (x) gewählt wird.

7. Verfahren nach Anspruch 6, wobei ein initialer latenter Zustand ($\tilde{x}_1$) der prognostizierten zeitlichen Entwicklung des latenten Zustands fest vorgegeben, insbesondere zufällig vorgegeben, wird oder wobei ein initialer latenter Zustand ($\tilde{x}_1$) aus der parametrierbaren Variationsfunktion (q(xi)) durch eine Verteilungsfunktion des initialen Zustands ($x_1$) gegeben den ermittelten Trainingsdatensatz ($q(x_1|y_{0:n}, u_{o:n})$) ermittelt wird, deren charakterisierende Parameter durch Rückpropagation trainierbar sind.

8. Verfahren nach einem der vorherigen Ansprüche, wobei eine optimale Steuergröße ($u_{opt}$) abhängig von einem mittels des Verfahrens ermittelten Verlaufs der Messgröße (y) ermittelt wird, wobei der Aktor (20) insbesondere mittels der optimalen Steuergröße ($u_{opt}$) angesteuert wird.

9. Verfahren zum Ermitteln von wenigstens einem optimalen Parameter ($\xi_{opt}$), der eine Regelstrategie eines Aktorsteuerungssystems (40) charakterisiert, welches eingerichtet ist, abhängig von dieser Regelstrategie einen Aktor (20) mit einer Steuergröße (u) anzusteuern,
wobei ein sich bei Anwendung der Regelstrategie ergebender zeitlicher Verlauf einer durch den Aktor (20) einstellbaren Messgröße (y) mittels des Verfahrens nach einem der Ansprüche 1 bis 8 ermittelt wird, und abhängig von dem so ermittelten Verlauf der Messgröße (y) der wenigstens eine optimale Parameter ($\xi_{opt}$) ermittelt wird.

10. Prognosesystem (60), welches eingerichtet ist, das Verfahren nach einem der Ansprüche 6 bis 7 auszuführen.

11. Aktorsteuerungssystem (40), welches eingerichtet ist, einen Aktor (20) mittels des Verfahren nach Anspruch 8 anzusteuern.

12. Verfahren zum Trainieren des Aktorsteuerungssystems (40) nach Anspruch 11, wobei Parameter ($\phi$) der parametrierbaren Familien von Funktionen ($q(x_{1:T}, f_{2:T}, z)$) und/oder deterministische Parameter derart angepasst werden, dass sie eine a-posteriori Wahrscheinlichkeitsverteilung $p(x_{1:T}, f_{2:T}, z \mid y_{1:T})$ von zumindest zeitlichen Verläufen mindestens des latenten Zustands des Aktors ($x_{1:T}$) und der Übergangsfunktion ($f_{2:T}$), bei einem gegebenen zeitlichen Verlauf der Messgröße ($y_{1:T}$) für einen ermittelten Trainingsdatensatz möglichst gut approximiert.

13. Maschinelles Trainingssystem (140), welches eingerichtet ist, das Aktorsteuerungssystem (40) nach Anspruch 11 mittels des Verfahrens nach Anspruch 12 zu trainieren.

14. Computerprogramm, welches eingerichtet ist, alle Schritte des Verfahrens nach einem der Ansprüche 1 bis 9 oder 12 auszuführen.

15. Maschinenlesbares Speichermedium (46, 2010), auf dem das Computerprogramm nach Anspruch 14 gespeichert ist.

**Claims**

1. Method for ascertaining a time characteristic of a measured variable (y) adjustable by an actuator (20), wherein a time characteristic of a control variable (u) is applied to the actuator (20),
wherein the ascertaining is effected by means of a Gaussian process state model of the behavior of the actuator (20), wherein the time characteristic of the measured variable (y) of the actuator (20) is ascertained on the basis of a parameterizable family of functions ($q(x_{1:T}, f_{2:T}, z)$), wherein in the parameterizable family of functions ($q(x_{1:T}, f_{2:T},$

z)) a time dependency of a later latent state ($x_t$), in particular ascertained using a transfer function ($f_t$), of the actuator (20) on an earlier latent state ($x_{t-1}$) of the actuator (20) and an earlier control variable ($u_{t-1}$) of the actuator (20) is the same as the applicable dependency of the Gaussian process state model. wherein the respective dependency means that the time dependencies within the parameterizable family of functions is maintained.

2. Method according to claim 1, wherein the parameterizable family of functions ($q(x_{1:T}, f_{2:T}, z)$) is set up to approximate an a-posteriori probability distribution ($p(x_{1:T}, f_{2:T}, z \mid y_{1:T})$) of at least time characteristics of at least the latent state ($x_{1:T}$) of the actuator (20) and the transfer function ($f_{2:T}$) as well as possible, at a given time characteristic of the measured variable ($y_{1:T}$) for an ascertained training data record ($y_{mess}$).

3. Method according to either claim 1 or claim 2, wherein the dependency of the parameterizable family of functions ($q(x_{1:T}, f_{2:T}, z)$) on an initial latent state ($x_1$) of the actuator (20) is given by a factor, which depends on this initial latent state ($x_1$), this factor being given by a parameterizable variation function ($q(x_1)$), in particular by a normal distribution ($N(m_{x1}, S_{x1})$).

4. Method according to any of claims 1 to 3, wherein the Gaussian process state model is a sparse Gaussian process state model with *inducing Gaussian process targets* ($z_1,...,z_P$) at predeterminable *pseudo input points* ($\zeta_1,...,\zeta_P$).

5. Method according to claims 3 and 4, wherein a dependency of the parameterizable family of functions ($q(x_{1:T}, f_{2:T}, z)$) on Gaussian process targets ($z_d$) is given by a second factor, wherein this second factor is a second parameterizable variation function ($q(z_d)$), which has the respective Gaussian process target ($z_d$) as an argument, wherein particularly the second parameterizable variation function ($q(z_d)$) is given by a normal distribution function ($N(z_d \mid m_d, S_d)$).

6. Method according to any of claims 4 to 5, wherein a predicted time trend of the latent state ($\tilde{x}_t$) of the actuator (20) is ascertained by recursively ascertaining a sample of the predicted time trend of the latent state at a later point in time ($\tilde{x}_{t+1}$) from the parameterizable variation function (q) of the predicted latent state at the later point in time given the predicted latent state at an earlier point in time ($q(\tilde{x}_{t+1}|\tilde{x}_t)$), the time characteristic of the measured variable (y) of the actuator (20) being chosen on the basis of the predicted time trend of the latent state (x).

7. Method according to claim 6, wherein an initial latent state ($\tilde{x}_1$) of the predicted time trend of the latent state is predetermined, in particular given randomly, or wherein an initial latent state ($\tilde{x}_1$) from the parameterizable variation function ($q(x_1)$) is ascertained by a distribution function of the initial state ($x_1$) given the ascertained training data record ($q(x_1|y_{0:n}, u_{0:n})$), the characterizing parameters of which can be trained by back propagation.

8. Method according to any of the preceding claims, wherein an optimal control variable ($u_{opt}$) is ascertained on the basis of a characteristic of the measured variable (y) ascertained by means of the method, wherein particularly the actuator (20) is controlled by means of the optimal control variable ($u_{opt}$).

9. Method for ascertaining at least one optimal parameter ($\xi_{opt}$) which characterizes a control strategy of an actuator control system (40), which is set up to control an actuator (20) with a control variable (u) on the basis of this control strategy, wherein, when using the control strategy, the time characteristic of a measurement variable (y) that is adjustable by the actuator (20) is ascertained by means of the method according to any of claims 1 to 8, and on the basis of the characteristic of the measured variable (y) thus ascertained, the at least one optimal parameter ($\xi_{opt}$) is ascertained.

10. Prediction system (60) set up to carry out the method according to any of claims 6 to 7.

11. Actuator control system (40) which is set up to control an actuator (20) by means of the method according to claim 8.

12. Method for training the actuator control system (40) according to claim 11, wherein parameters ($\phi$) of the parameterizable families of functions ($q(x_{1:T}, f_{2:T}, z)$) and/or deterministic parameters are adapted such that they approximate an a-posteriori probability distribution $p(x_{1:T}, f_{2:T}, z \mid y_{1:T})$ of at least time characteristics of at least the latent state of the actuator ($x_{1:T}$) and the transfer function ($f_{2:T}$) as well as possible, at a given time characteristic of the measured variable ($y_{1:T}$) for an ascertained training data record.

13. Machine training system (140) which is set up to train the actuator control system (40) according to claim 11 by means of the method according to claim 12.

**14.** Computer program which is set up to carry out all steps of the method according to any of claims 1 to 9, or 12.

**15.** Machine-readable storage medium (46, 2010) storing the computer program according to claim 14.

**Revendications**

**1.** Procédé permettant la détermination d'un profil temporel d'une grandeur de mesure (y) qui peut être réglée par un actionneur (20), un profil temporel d'une grandeur de commande (u) étant appliqué à l'actionneur (20), la détermination du comportement de l'actionneur (20) étant effectuée au moyen d'un modèle d'état de processus gaussien, le profil temporel de la grandeur de mesure (y) de l'actionneur (20) étant déterminé en fonction d'une famille paramétrable de fonctions (q($x_{1:T}$, $f_{2:T}$, z)), une dépendance temporelle d'un état ($x_t$) de l'actionneur (20) déterminé en particulier par une fonction de transition (ft), latent et temporellement ultérieur d'un état latent temporellement antérieur ($x_{t-1}$) de l'actionneur (20) et d'une grandeur de commande temporellement antérieure ($u_{t-1}$) de l'actionneur (20) étant égale à la dépendance correspondante du modèle d'état de processus gaussien dans la famille paramétrable de fonctions (q($x_{1:T}$, $f_{2:T}$, z)), la dépendance correspondante signifiant que la dépendance temporelle est conservée dans la famille paramétrable de fonctions.

**2.** Procédé selon la revendication 1, dans lequel la famille paramétrable de fonctions (q($x_{1:T}$, $f_{2:T}$, z)) est configurée pour approximer aussi bien que possible une distribution de probabilité à postériori (p($x_{1:T}$, $f_{2:T}$, z | $y_{1:T}$)) d'au moins des profils temporels au moins de l'état latent ($x_{1:T}$) de l'actionneur (20) et de la fonction de transition ($f_{2:T}$), lors d'un profil temporel donné de la grandeur de mesure ($y_{1:T}$) pour un ensemble de données d'apprentissage déterminé ($y_{mess}$).

**3.** Procédé selon la revendication 1 ou 2, dans lequel la dépendance de la famille paramétrable de fonctions (q($x_{1:T}$, $f_{2:T}$, z)) d'un état latent initial ($x_1$) de l'actionneur (20) est donnée par un facteur qui dépend dudit état latent initial ($x_1$), ledit facteur étant donné par une fonction de variation paramétrable (q(xi)), en particulier par une distribution normale (N($m_{x1}$, $S_{x1}$)).

**4.** Procédé selon l'une des revendications 1 à 3, dans lequel le modèle d'état de processus gaussien est un modèle d'état de processus gaussien clairsemé comportant des états cibles de processus gaussien inducteurs (*inducing Gaussian process targets* en anglais) ($z_1$,...,$Z_P$) au niveau de pseudo points d'entrée spécifiables (*pseudo input points* en anglais) ($\zeta_1$,..., $\zeta_P$).

**5.** Procédé selon les revendications 3 et 4, dans lequel une dépendance de la famille paramétrable de fonctions (q($x_{1:T}$, $f_{2:T}$, z)) d'états cibles de processus gaussien ($z_d$) est respectivement donnée par un second facteur, ledit second facteur étant respectivement une seconde fonction de variation paramétrable (q($z_d$)) qui possède l'état cible du processus gaussien ($z_d$) respectif comme argument, la seconde fonction de variation paramétrable (q($z_d$)) en particulier étant donnée par une fonction de distribution normale (N($z_d$ | $m_d$, $S_d$)).

**6.** Procédé selon l'une des revendications 4 à 5, dans lequel une évolution temporelle prédite de l'état latent ($\tilde{x}_t$) de l'actionneur (20) est déterminée en déterminant, de manière récursive, un échantillon de l'évolution temporelle prédite de l'état latent à un instant ultérieur ($\tilde{x}_{t+1}$) à partir de la fonction de variation paramétrable (q) de l'état latent prédit à un instant ultérieur en fonction de l'état latent prédit à un instant antérieur (q($\tilde{x}_{t+1}$ | $\tilde{x}_t$)), le profil temporel de la grandeur de mesure (y) de l'actionneur (20) étant choisi en fonction de l'évolution temporelle prédite de l'état latent (x).

**7.** Procédé selon la revendication 6, dans lequel un état latent initial ($\tilde{x}_1$) de l'évolution temporelle prédite de l'état latent est spécifié en détail, en particulier aléatoirement, ou dans lequel un état latent initial ($\tilde{x}_1$) est déterminé à partir de la fonction de variation paramétrable (q(xi)) par une fonction de distribution de l'état initial ($x_1$) en fonction de l'ensemble de données d'apprentissage déterminé (q($x_1$ | $y_{0:n}$, $u_{0:n}$)), dont les paramètres caractéristiques peuvent être appris par rétropropagation.

**8.** Procédé selon l'une des revendications précédentes, dans lequel une grandeur de commande optimale ($u_{opt}$) est déterminée en fonction d'un profil temporel de la grandeur de mesure (y) déterminé au moyen du procédé, l'actionneur (20) étant activé en particulier au moyen de la grandeur de commande optimale ($u_{opt}$).

**9.** Procédé permettant la détermination d'au moins un paramètre optimal ($\xi_{opt}$) qui caractérise une stratégie de réglage d'un système de commande d'actionneur (40), lequel est configuré pour activer un actionneur (20) comportant une grandeur de commande (u) en fonction de ladite stratégie de réglage,

dans lequel un profil temporel, résultant de l'application de la stratégie de réglage, d'une grandeur de mesure (y) réglable par l'actionneur (20) est déterminé au moyen du procédé selon l'une des revendications 1 à 8 et l'au moins un paramètre optimal ($\xi_{opt}$) est déterminé en fonction du profil ainsi déterminé de la grandeur de mesure (y).

10. Système de prédiction (60) configuré pour exécuter le procédé selon l'une des revendications 6 à 7.

11. Système de commande d'actionneur (40) configuré pour activer un actionneur (20) au moyen du procédé selon la revendication 8.

12. Procédé permettant l'apprentissage du système de commande d'actionneur (40) selon la revendication 11, dans lequel des paramètres ($\phi$) des familles paramétrables de fonctions ($q(x_{1:T}, f_{2:T}, z)$) et/ou des paramètres déterministes sont adaptés de telle sorte qu'ils approximent aussi bien que possible une distribution de probabilité à posteriori $p(x_{1:T}, f_{2:T}, z \mid y_{1:T})$ d'au moins des profils temporels au moins de l'état latent de l'actionneur ($x_{1:T}$) et de la fonction de transition ($f_{2:T}$), lors d'un profil temporel donné de la grandeur de mesure ($y_{1:T}$), pour un ensemble de données d'apprentissage déterminé.

13. Système d'apprentissage automatisé (140) configuré pour permettre l'apprentissage du système de commande d'actionneur (40) selon la revendication 11 au moyen du procédé selon la revendication 12.

14. Programme informatique configuré pour exécuter toutes les étapes du procédé selon l'une des revendications 1 à 9 ou 12.

15. Support de stockage lisible par machine (46, 2010) sur lequel est stocké le programme informatique selon la revendication 14.

Fig. 1

Fig. 2

```
┌─────────┐
│  1000   │
└────┬────┘
     │
     ▼
┌─────────┐
│  1100   │
└────┬────┘
     │
     ▼
┌─────────┐
│  1150   │◄──────────────────┐
└────┬────┘                   │
     │                        │
     ▼                        │
┌─────────┐                   │
│  1200   │                   │
└────┬────┘                   │
     │                        │
     ▼                        │
┌─────────┐     ◇◇◇◇◇◇◇       ┌─────────┐
│  1300   │──►◇  1400  ◇──►│  1500   │
└─────────┘     ◇◇◇◇◇◇◇       └─────────┘
```

**Fig. 3**

2000 → 2010 → 2020 → 2030 → 2040

2060 → 2020

2040 → 2050

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 202017102235 U1

**In der Beschreibung aufgeführte Nicht-Patentliteratur**

- **ROGER FRIGOLA ; YUTIAN CHAN ; CARL E. RASMUSSEN.** Variational Gaussian Process State-Space Models. *arXiv preprint arXiv:1406.4905v2,* 2014

- **STEFANOS ELEFTHERIADIS ; THOMAS F.W. NICHOLSON ; MARC PETER DEISENROTH.** Identification of Gaussian Process State Space Models. *arXiv preprint arXiv: 1705.10888v2,* 2017
- **JAMES HENSMAN.** A flexible state space model for learning nonlinear dynamical systems. *arXiv preprint arXiv:1603.05486v2,* 2017